Europäisches Patentamt

(19) **European Patent Office**

**Office européen des brevets**

(11) Numéro de publication: **0 079 306**
**A2**

## (12) DEMANDE DE BREVET EUROPEEN

(21) Numéro de dépôt: **82810447.1**

(22) Date de dépôt: **26.10.82**

(51) Int. Cl.³: **A 61 B 5/02**

(30) Priorité: **05.11.81 DE 3143871**

(43) Date de publication de la demande:
**18.05.83 Bulletin 83/20**

(84) Etats contractants désignés:
**CH DE FR GB LI**

(71) Demandeur: **ASULAB S.A.**
**Faubourg du Lac 6**
**CH-2501 Bienne(CH)**

(72) Inventeur: **Klaye, François**
**Port-Roulant 12A**
**CH-2000 Neuchatel(CH)**

(74) Mandataire: **de Montmollin, Henri et al,**
**c/o ASUAG - Société Générale de l'Horlogerie Suisse**
**S.A. 6, Faubourg du Lac**
**CH-2501 Bienne(CH)**

(54) **Procédé de mesure de la pression sanguine d'un sujet.**

(57) L'invention concerne un procédé de mesure de la pression sanguine d'un sujet à l'aide d'un sphygmomanomètre ayant un brassard (1) muni d'une chambre déformable (3) et d'un microphone (5).

Après fixation du brassard (1) sur le bras du sujet et gonflage de la chambre (3) à une pression suffisante, on fait décroître cette pression de façon discontinue par pas de décompressions ayant tous une même amplitude $\Delta p$. Dans une première phase où aucun son ayant une intensité supérieure à un premier seuil $W_o$ ne peut être détecté à l'aide du microphone (5), on effectue les pas de décompression à intervalles réguliers, toutes les 1,6 secondes par exemple. Dans une deuxième phase qui commence après la détection de deux premiers sons $(S_4, S_5)$ dont l'intensité dépasse ledit seuil $W_o$, on effectue un pas de décompression à chaque apparition d'un nouveau son $S_{oi}$ dont l'intensité dépasse un seuil $W_i$ associé audit son et déterminé en fonction de l'intensité d'au moins un des sons détectés précédemment.

./...

Fig. 9

## PROCEDE DE MESURE DE LA PRESSION SANGUINE D'UN SUJET

La présente invention concerne un procédé de mesure de la pression sanguine d'un sujet à l'aide d'un sphygmomanomètre ayant un brassard fixé sur un membre, par exemple un bras, dudit sujet et comportant une chambre déformable contenant un gaz sous pression.

Habituellement, après avoir fixé le brassard sur le bras du sujet et après avoir fait monter la pression de gaz régnant dans la chambre jusqu'à une valeur maximale supérieure à la pression systolique attendue du sujet, on mesure la pression sanguine du sujet de la façon suivante : on fait décroître cette pression et, durant cette décroissance de pression, on analyse les conditions de circulation du sang dans l'artère traversant ledit bras du sujet, par exemple en détectant les sons émis par ladite artère, et on mesure la pression régnant dans ladite chambre déformable à deux moments à chacun desquels on observe un changement dans ces conditions de circulation. Ces moments sont par exemple, respectivement, celui où ladite artère commence à émettre des sons appelés communément sons de Korotkoff, et celui où ladite artère cesse d'émettre ces sons.

Le brevet américain no 4.144.879 décrit un procédé de mesures de la pression sanguine d'un sujet selon lequel, au lieu de détecter les sons de Korotkoff pour la détermination de ces deux moments de changement de conditions de circulation sanguine, on utilise des signaux obtenus à partir de la deuxième dérivée d'un signal représentatif de la pression dans la chambre déformable. De plus, selon ce procédé connu, on fait décroître de façon continue la pression de gaz régnant dans cette chambre déformable jusqu'à l'apparition du premier changement dans lesdites conditions de circulation sanguine. A la suite de ce premier changement, la pression régnant dans ladite chambre déformable est diminuée de façon discontinue en effectuant des décompressions espacées les unes des autres dans le temps. Chacune de ces décompressions coïncide avec un battement du pouls du sujet.

Néanmoins, selon ce procédé connu, chacune de ces décompressions est obtenue en maintenant ouverte une vanne d'échappement pendant une durée prédéterminée qui est la même pour toutes les décompressions, de sorte que ces décompressions ne présentent pas toutes la même amplitude, mais, au contraire une amplitude décroissant depuis la première jusqu'à la dernière décompression.

La précision de la mesure de pression au premier et au second desdits moments étant une fonction de la vitesse de décroissance de la pression régnant dans ladite chambre déformable, il en résulte que, dans ce procédé connu, on mesure les deux pressions régnant dans la chambre respectivement au premier et au second moment avec des précisions différentes.

Un autre inconvénient de ce procédé connu réside dans le fait que la décroissance de la pression dans la chambre déformable n'est pas réalisée selon un mode unique mais, au contraire, selon deux modes de décompression différents à savoir, un premier mode selon lequel la pression dans la chambre déformable diminue de façon continue et un second mode selon lequel cette pression diminue de façon discontinue par décompressions espacées dans le temps les unes des autres. Pour une mise en oeuvre de ces deux modes différents, il est nécessaire d'utiliser deux vannes différentes, car le premier mode nécessite l'utilisation d'une vanne dont l'ouverture doit être réglée à une valeur bien plus faible que celle que doit présenter la vanne permettant la mise en oeuvre du second mode.

Il en résulte que ce procédé connu nécessite l'utilisation d'un matériel coûteux.

C'est pourquoi, la présente invention à notamment pour objet de proposer un procédé permettant l'utilisation d'une seule vanne de grande ouverture fonctionnant par tout ou rien.

Un deuxième objet de la présente invention est de proposer un procédé permettant de mesurer la pression régnant dans la chambre à chacun desdits moments précités, avec une précision identique pour les deux moments et ne dépendant pas de la valeur de cette pression.

A ces effets, le procédé de l'invention consiste à faire décroître la pression régnant dans la chambre déformable de façon discontinue depuis sa valeur maximale jusqu'à une valeur finale, en effectuant des décompressions d'amplitude constante et espacées dans le temps les unes des autres.

Ce procédé permet de n'utiliser qu'une vanne d'échappement qui, de surcroît est de faible coût puisqu'elle est du type fonctionnant par tout ou rien. De plus, ce procédé permet d'effectuer les mesures de pression systolique et diastolique avec une précision identique.

Selon un mode de réalisation actuellement préféré, les conditions de circulation sanguine dans ladite artère du sujet sont analysées en détectant les sons émis par cette artère dont l'intensité est supérieure à un certain seuil, et lesdites décompressions sont effectuées de la façon suivante :

- pendant une première phase de décroissance de la pression régnant dans la chambre déformable, phase durant laquelle lesdits sons sont absents, lesdites décompressions sont effectuées à des instants espacés par des intervalles de temps constants, par exemple de 1,6 secondes, et

- pendant une seconde phase de ladite décroissance de pression, phase durant laquelle lesdits sons sont présents, une décompression est effectuée à l'apparition de chacun desdits sons.

Les caractéristiques et avantages de l'invention seront mieux compris à la lecture de la description qui va suivre d'un mode de réalisation, description faite en référence aux dessins annexés dans lequels :

- la figure 1 est un schéma synoptique d'un sphygmomanomètre selon un mode de réalisation de l'invention;

- les figures 2 et 3 représentent le diagramme du programme du micro-ordinateur utilisé par le sphygmomanomètre de la figure 1;

- la figure 4 représente le diagramme détaillé du sous-programme "$K_{oi}$?" utilisé dans le programme des figures 2 et 3;

- la figure 5 est le diagramme détaillé du sous-programme "décompression d'un pas" utilisé dans le programme des figures 2,3 et 7;

- la figure 6 représente le diagramme détaillé du sous-programme "validation premier K" utilisé dans le programme des figures 2 et 3;

- la figure 7 représente le diagramme détaillé du sous-programme "validation K" utilisé dans le programme des figures 2 et 3;

- la figure 8 représente le diagramme détaillé du sous-programme "calcul et mise en mémoire du seuil" utilisé dans le programme des figures 2 et 3;

- la figure 9 représente le diagramme en fonction du temps de la pression régnant dans la chambre déformable du brassard du sphygmomanomètre de la figure 1, au cours d'un cycle de mesure, ainsi que les sons détectés au cours de ce cycle; et

- la figure 10 représente une portion finale d'un diagramme analogue à celui de la figure 9, illustrant le cas où un son parasite survient au moment où les sons de Korotkoff deviennent inférieurs au seuil de détection.

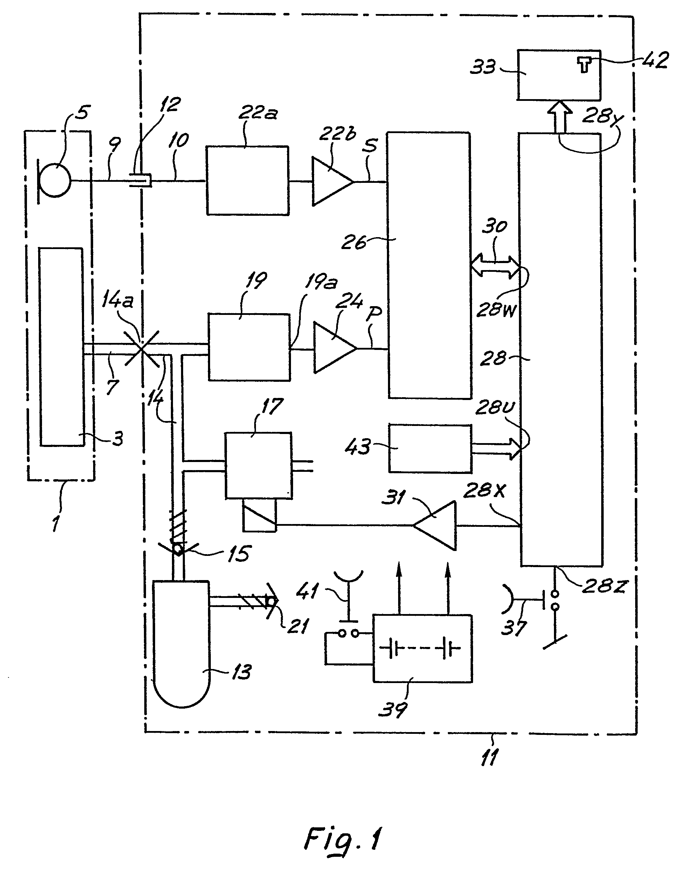

Le sphygmomanomètre représenté à titre d'exemple à la figure 1 comporte un brassard 1 destiné à être fixé sur le bras d'un sujet à examiner, et un appareil désigné dans son ensemble par la référence 11. Le brassard 1 comprend une chambre déformable gonflable 3, par exemple à parois en caoutchouc, et un microphone 5. La chambre 3 est reliée à l'appareil 11 par une conduite d'air souple 7 qui est elle-même reliée de façon amovible à une conduite 14 de l'appareil 11 par une prise d'air 14a. Le microphone 5 est relié à l'appareil 11 par un câble électrique souple 9 qui est relié de façon amovible à un conducteur 10 de l'appareil 11 par un connecteur 12.

L'appareil 11 comporte une source d'énergie électrique 39, par exemple une batterie rechargeable ou une pile, destinée à assurer l'alimentation électrique de ses différentes parties. Cette source 39 est munie d'un interrupteur 41 pour sa mise en et hors service.

L'appareil 11 comprend en outre une pompe qui dans l'exemple représenté est une pompe manuelle 13 formée d'un corps creux en caoutchouc de forme cylindrique. La chambre 3 est reliée à la pompe 13 par la conduite 7, la prise d'air 14a et la conduite 14 sur laquelle est interposée une valve anti-retour 15 ouvrant dans le sens allant de la pompe 13 à la chambre 3. La chambre 3 est également reliée, par la conduite 7 et par une première partie de la conduite 14, à une vanne de décompression à commande électrique 17 ainsi qu'à un capteur de pression 19. La pompe 13 est en outre munie d'une entrée d'air comportant une valve anti-retour 21 ouvrant dans le sens allant de l'extérieur vers l'intérieur de la pompe 13.

Ainsi, par une pression manuelle suivie d'un relâchement de la pompe 13, on aspire de l'air à l'extérieur et on introduit cet air dans la chambre 3.

Le microphone 5 est relié électriquement par le câble 9, le connecteur 12 et le conducteur 10 à un circuit de filtrage 22a suivi d'un circuit amplificateur 22b. L'ensemble formé par le microphone 5, le circuit de filtrage 22a et le circuit amplificateur 22b constitue un dispositif répondant à tout son émis dans une zone contenant le brassard 1 en fournissant un signal de son S représentatif de l'intensité dudit son.

Le capteur de pression 19, qui est par exemple constitué par un montage électrique en pont d'éléments piézo-résistifs, fournit sur sa sortie 19a un signal de tension dont l'amplitude est proportionnelle à la pression p de gaz régnant dans la chambre 3. Ce signal est amplifié dans le circuit amplificateur 24.

Le signal de son S fourni par l'amplificateur 22b et le signal de mesure de pression P fourni par l'amplificateur 24 sont appliqués sur des entrées respectives d'un circuit 26 ayant pour fonction d'effectuer une conversion analogique - numérique de ces signaux S et P et de fournir à un micro-ordinateur 28 tantôt le signal numérique correspondant au signal S, tantôt le signal numérique correspondant au signal P selon l'état d'un signal de commande qui lui est fourni par ledit micro-ordinateur 28. Ces signaux numériques ainsi que ledit signal de commande sont transmis par un ensemble de conducteurs 30, généralement appelé bus bi-directionnel. Le circuit 26 est constitué par exemple par un circuit intégré commercialisé par la société NIPPON ELECTRIC CO. (Japon) sous la référence commerciale μ PD 7001. Le circuit 28 est, lui, constitué par exemple par un circuit intégré commercialisé par la société précitée sous la référence commerciale μ PD 7502. Une sortie 28X du circuit 28 est reliée, par l'intermédiaire d'un amplificateur 31, à l'électroaimant de commande de la vanne 17.

Comme cela sera expliqué plus en détail ci-dessous, un signal logique à deux niveaux apparaît sur la sortie 28 X du circuit 28 lors du fonctionnement de l'appareil 11. L'amplificateur 31 et l'électrovanne 17 sont conçus de telle sorte que chacun desdits niveaux corresponde à la mise dans un état complètement ouvert respectivement complètement fermé de la vanne 17.

D'autres sorties 28Y du circuit 28 sont reliées à un dispositif d'affichage 33, par exemple un dispositif à cristal liquide, destiné à afficher les valeurs numériques des pressions systolique et diastolique du sujet et, le cas échéant, celle du pouls du sujet, ainsi que des symboles destinés à informer l'utilisateur de l'appareil des états de fonctionnement dans lesquels peut se trouver ce dernier. Le circuit 28 possède également une borne 28Z destinée à recevoir un signal de mise en conditions de fonctionnement initiales fourni par l'actionnement d'un interrupteur fugitif à bouton-poussoir 37.

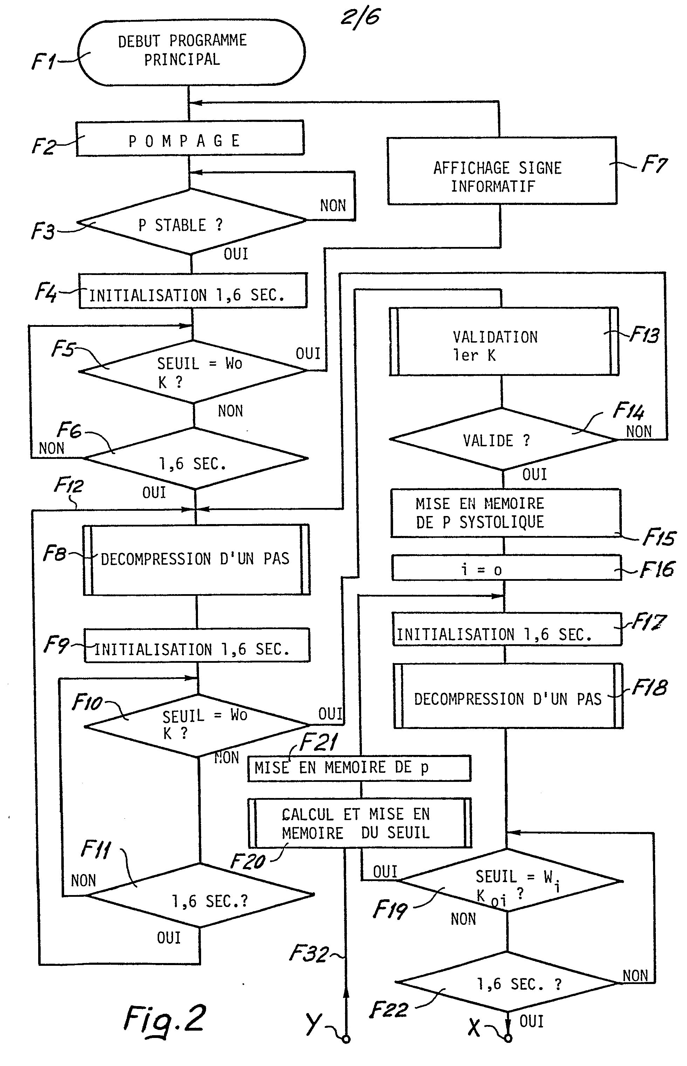

Le fonctionnement du sphygmomanomètre représenté sur la figure 1 va maintenant être décrit en faisant référence aux figures 2 à 10.

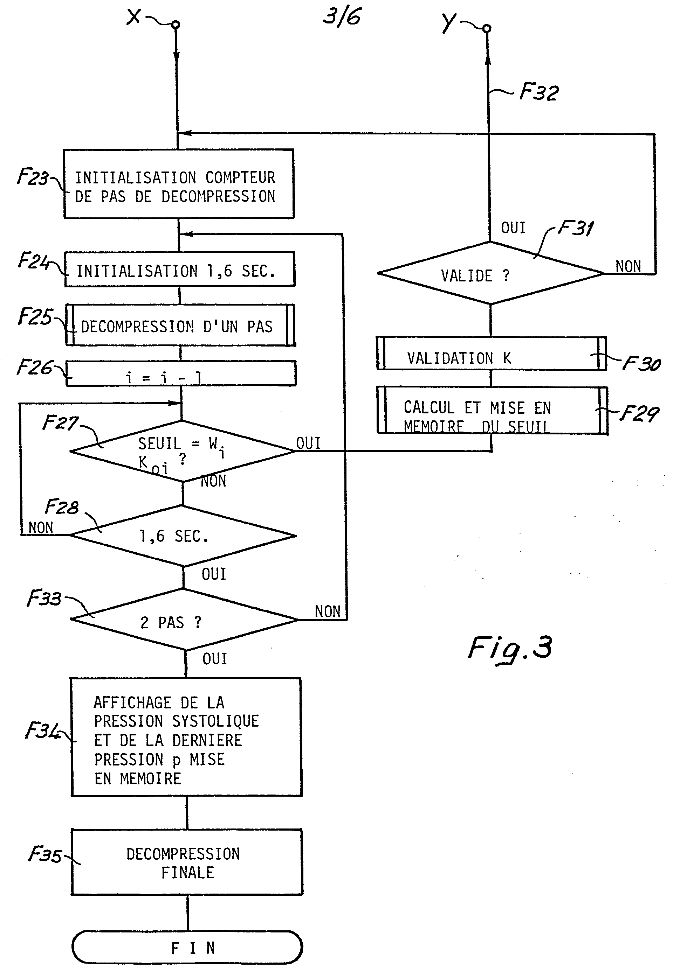

Le micro-ordinateur 28 est programmé de manière à fontionner selon le programme représenté sur les figures 2 et 3. L'étape F1 de ce programme correspond à la mise en service de l'appareil 11 en réponse à la fermeture l'interrupteur 41 et à l'actionnement de l'interrupteur 37. Cette mise en service comporte l'accomplissement d'un sous-programme de recalage à zéro de la mesure de la pression régnant dans la chambre 3. Ce sous-programme ne faisant pas l'objet de la présente invention, il ne sera pas décrit ici plus en détail.

L'étape F2 du programme concerne la mise sous pression de la chambre 3 par l'utilisateur à l'aide de la pompe manuelle 13. Cette étape F2 ne constitue donc pas à proprement parler une étape du programme introduit dans le micro-ordinateur 28.

L'étape F3 suspend la poursuite ultérieure du programme jusqu'à ce que la pression p régnant dans la chambre 3 devienne stable.

La partie de programme suivante F4 à F7 permet l'affichage par le dispositif 33 d'un symbole informatif spécial 42 (voir figure 1) informant l'utilisateur de l'appareil que la pression régnant dans la chambre 3 est encore insuffisante et qu'il doit pomper à nouveau. La partie de programme F4 à F7 consiste à déterminer si, durant un laps de temps de, par exemple, 1,6 secondes de durée suivant immédiatement la stabilisation de la pression, un signal S d'amplitude supérieure à une valeur initiale de seuil $W_o$ apparaît ou non à la sortie de l'amplificateur 22b. La valeur initiale de seuil $W_O$ est une donnée constante mais réglable.

Dans le cas où aucun signal S satisfaisant à cette condition n'est émis par l'amplificateur 22b durant ce laps de temps, c'est-à-dire dans le cas où la pression régnant dans la chambre 3 a dépassé la pression systolique du sujet, aucun symbole informatif 42 n'est affiché, l'utilisateur cesse de pomper et le programme se poursuit par l'accomplissement des étapes suivantes F8 à F11.

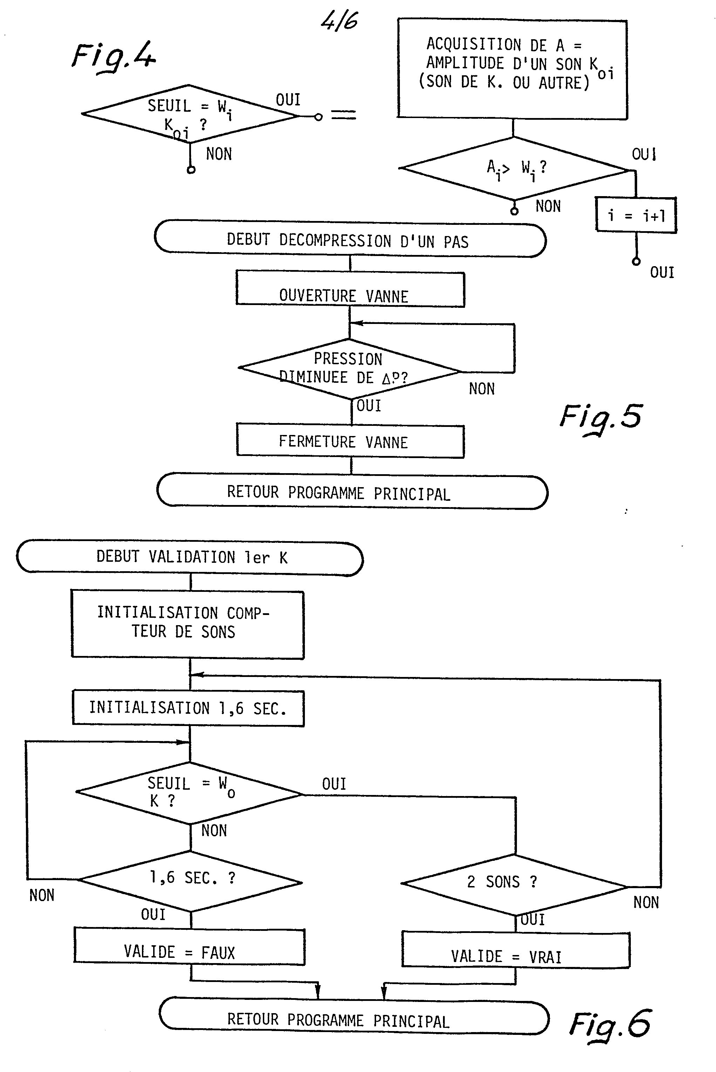

L'étape F8 est une étape de décompression de la chambre 3 d'une quantité $\Delta p$ constante mais réglable. Au début de cette étape de décompression F8, qui est illustrée plus en détail par la figure 5, la valeur $P_O$ du signal P correspondant à la valeur $p_O$ de la pression dans la chambre 3 est mise en mémoire, et le micro-ordinateur 28 délivre sur sa sortie 28X un signal qui provoque l'ouverture de la vanne de décompression 17. La pression dans la chambre 3 et le signal P commencent à diminuer. Le micro-ordinateur 28 compare ce signal P à la valeur mémorisée $P_O$ et il provoque la fermeture de la vanne 17 dès que ce signal devient égal à la différence $P_O - \Delta P$, où $\Delta P$ est la tension qui correspond à la différence de pression $\Delta p$ désirée.

Les données correspondant à la valeur de seuil $W_O$ et à la quantité $\Delta P$ ont préalablement été introduites sous forme numérique par les entrés 28U, dans le circuit 28, au moyen d'un dispositif d'entrée de données 43 se trouvant à l'intérieur d'un boîtier non représenté contenant les organes électroniques de l'appareil 11. Le dispositif 43 permet aussi de modifier à volonté les valeurs $W_O$ et $\Delta P$, afin notamment d'adapter ces paramètres aux caractéristiques personnelles du sujet.

Le dispositif 43 est par exemple constitué par un ensemble de commutateurs manoeuvrables manuellement, pouvant chacun commuter soit le niveau logique "0", soit le niveau logique "1" sur une entrée 28U respective du micro-ordinateur 28.

Les étapes F9 à F11 ont pour fonction de déterminer si pendant un laps de temps ayant une durée déterminée de, par exemple, 1,6 secondes suivant une décompression d'un pas (étape F8), un signal S d'amplitude supérieure au seuil $W_o$ est fourni par l'amplificateur 22b. La sortie OUI de l'étape d'interrogation F11 étant reliée à l'entrée de l'étape F8 par l'étape de liaison F12, on voit que les étapes F8 à F12 réalisent un premier processus de décroissance discontinue de la pression p régnant dans la chambre 3. Pendant ce processus, la pression p décroît par une succession de décompressions de même amplitude $\Delta p$, ces décompressions étant régulièrement espacées dans le temps, avec des intervalles égaux à 1,6 secondes dans le présent exemple. Ce premier processus de décompression se poursuit tant que l'amplificateur 22b ne fournit pas de signal S d'amplitude supérieure au seuil $W_o$.

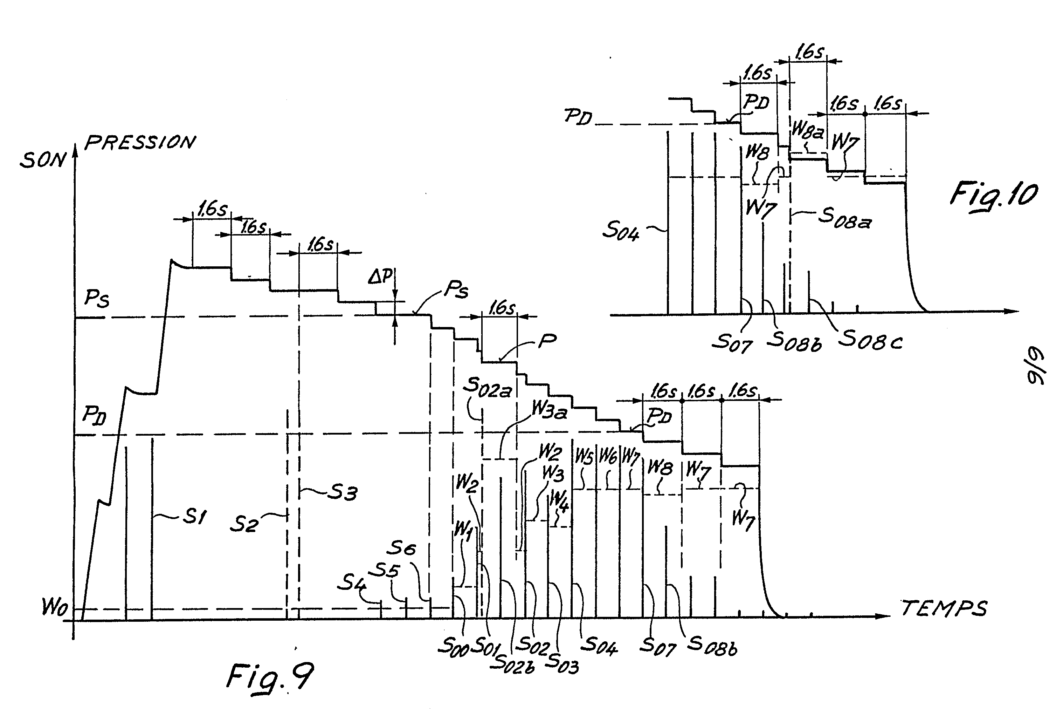

Dès qu'un signal S d'amplitude supérieure à $W_o$ est fourni par l'amplificateur 22b, le processus de décompression est interrompu et le programme se poursuit par l'accomplissement du sous-programme F13, F14 dit de validation d'un premier son de Korotkoff. La figure 6 représente de façon détaillée ce sous-programme F13, F14, qui consiste à déterminer si ce premier signal S fourni par l'amplificateur 22b est suivi ou non par deux autres signaux S disposés de telle sorte que ces trois signaux S se succèdent les uns aux autres à des intervalles de temps inférieurs à 1,6 secondes. En se reportant à la figure 9, on voit que le signal S2 est suivi par un seul autre signal S3. Ce signal S2 ne correspond donc pas à un son de Korotkoff, mais est dû à un son parasite. Dans ce cas, l'étape F14 produit une réponse NON qui entraîne un retour à la boucle de programme F8 à F12.

Sur la figure 9 on voit que le signal S4, au contraire, est bien suivi de deux autres signaux S5 et S6, ces trois signaux S4 à S6 ayant une amplitude supérieure à $W_0$ et se succédant à des intervalles de temps inférieurs à 1,6 secondes. Ce signal S4 correspond donc bien au premier son de Korotkoff. Dans ce cas, le sous-programme F13, F14 produit un OUI à l'étape d'interrogation F14, ce qui entraîne la poursuite du programme par l'accomplissement de l'étape F15 de mise en mémoire de la valeur $P_S$ de la pression dans la chambre 3 à cet instant. Cette valeur $P_S$ est celle de la pression systolique du sujet.

Les étapes suivantes F16 à F19 consistent à effectuer une décompression de la chambre 3 de la quantité $\Delta p$ à l'apparition du troisième signal S6, et à déterminer si, à la suite de cette décompression, il apparaît à la sortie de l'amplificateur 22b un nouveau signal S dont l'amplitude dépasse également le seuil $W_0$. L'étape de décompression F18 est identique à l'étape F8 décrite ci-dessus.

Si, comme dans le cas illustré par la figure 9, un nouveau signal $S_{00}$ ayant une amplitude supérieure au seuil $W_0$ apparaît moins de 1,6 secondes après l'apparition du signal S6, une nouvelle valeur de seuil $W_1$ est déterminée (étape F20), la pression p dans la chambre 3 est mémorisée (étape F21) et une nouvelle décompression de la chambre 3 de la quantité $\Delta p$ est effectuée (étape F18). La sortie OUI de l'étape d'interrogation F19 étant reliée à l'entrée de l'étape F17 par les étapes F20 et F21, on voit que les étapes F17 à F21 forment avec l'étape F22 qui est liée fonctionnellement à l'étape F19, une boucle de programme déterminant un second processus de décroissance de la pression régnant dans la chambre 3. Pendant ce second processus, une décompression d'amplitude $\Delta p$ est effectuée si un signal $S_{oi}$ ayant une amplitude supérieure à un seuil $W_i$ associé à ce signal apparaît moins de 1,6 secondes après l'apparition du signal précédent $S_{oi-1}$. Plus généralement, à chaque cycle d'indice i de la boucle de programme F17 à F22 du micro-ordinateur 28, on valide un signal S fourni par l'amplificateur 22b et dont l'amplitude dépasse un seuil de détection $W_i$ déterminé au cours du cycle précédent d'indice (i-1). A l'apparition de ce signal $S_{oi}$ ainsi validé, on détermine une nouvelle valeur de seuil $W_{i+1}$ qui servira pour valider un signal $S_{oi+1}$ suivant, on mémorise la pression p régnant dans la chambre 3 et on effectue une nouvelle décompression de la chambre 3 de la quantité $\Delta p$.

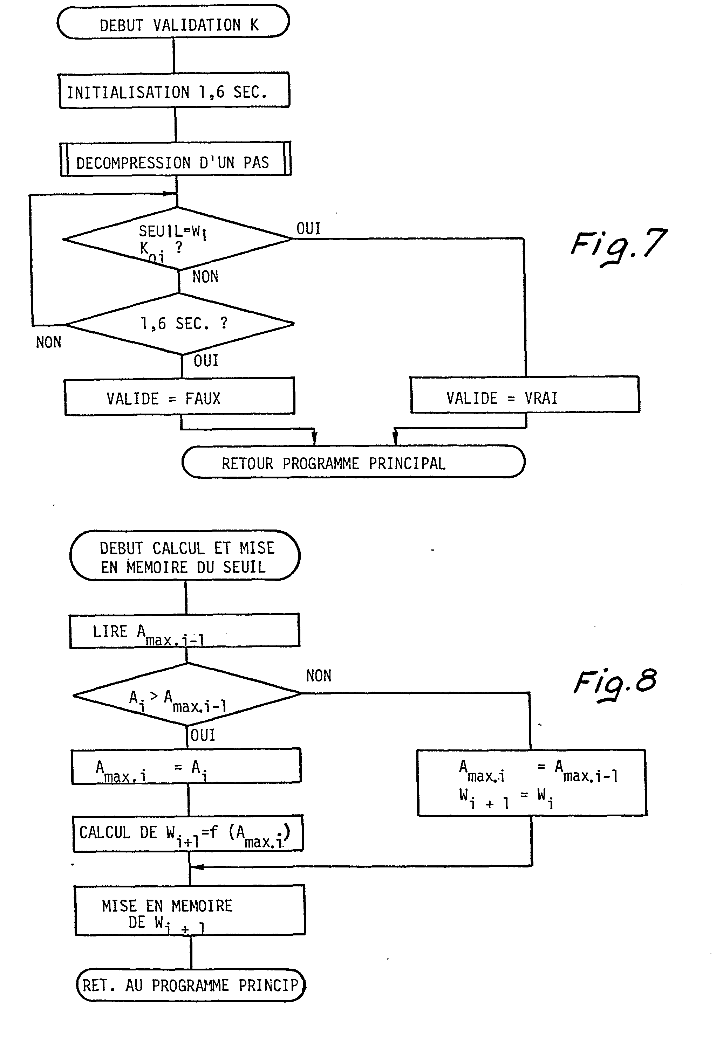

L'étape F20 de calcul et de mise en mémoire du seuil consiste en un sous-programme représenté plus en détail à la figure 8. Ce sous-programme consiste à déterminer à chaque apparition d'un signal validé $S_{oi}$ une nouvelle valeur de seuil $W_{i+1}$ devant servir pour la validation d'un signal $S_{oi+1}$ suivant. Cette valeur de seuil $W_{i+1}$ est une fonction croissante f d'une grandeur $G_i$ qui est elle-même fonction de l'amplitude d'au moins un signal S validé antérieurement. Par exemple, on peut choisir pour $W_{i+1}$ une valeur proportionnelle à la grandeur $G_i$, avec un coefficient de proportionnalité $\alpha < 1$. La valeur du coefficient $\alpha$, 0,75 par exemple, peut être introduite, de manière non modifiable, dans le programme du micro-ordinateur. Elle peut également être introduite au moyen d'un dispositif d'entrée de données semblable au dispositif 43 décrit ci-dessus. Dans ce cas, la personne utilisant l'appareil peut modifier cette valeur $\alpha$ à volonté.

Selon un mode possible de calcul du seuil $W_{i+1}$, la grandeur $G_i$ est égale à l'amplitude $A_i$ dudit signal $S_{oi}$.

On préfère cependant utiliser un autre mode de calcul de $W_{i+1}$ illustré par le diagramme de la figure 8, selon lequel on détermine $W_{i+1}$ de la façon suivante :

A chaque validation d'un signal $S_{oi}$, l'amplitude $A_{max.i-1}$ du plus grand des signaux $S_{oo}$ à $S_{oi-1}$ validés au cours des cycles de programme précédents est comparée à la valeur $A_i$ de l'amplitude du signal $S_{oi}$.

Si l'on a $A_i > A_{max.i-1}$, on donne au nouveau seuil $W_{i+1}$ la valeur $f(A_i)$, f étant la fonction croissante mentionnée ci-dessus.

Si, au contraire, on a $A_i \leq A_{max.i-1}$, on donne au seuil $W_{i+1}$ sa valeur déterminée précédemment $W_i$.

Comme cela sera expliqué ci-dessous, la boucle de programme F22 à F31 des figures 2 et 3 permet d'identifier comme signal parasite $S_a$ tout signal validé S remplissant les conditions suivantes :

1) ce signal $S_a$ entraîne la fixation du seuil à une valeur $W_{(i+1)a}$ non seulement supérieure à la valeur $W_i$ du seuil précédent mais aussi trop élevée pour permettre la validation d'un nouveau signal S dans un délai de 1,6 secondes; et

2) au cours d'un cycle suivant commençant 1,6 secondes après l'apparition du signal $S_a$, l'utilisation de la valeur de seuil précédente $W_i$ permet de valider un nouveau signal S dans un délai de 1,6 secondes commençant au début de ce cycle.

Il a été dit plus haut que, selon un mode de réalisation préféré, la grandeur $G_i$ servant au calcul du seuil $W_{i+1}$ est l'amplitude $A_{max.i}$ du plus grand des signaux $S_{oo}$ à $S_{oi}$ validés précédemment. Il faut noter que si un signal parasite $S_a$ est validé au cours d'un cycle de programme antérieur au cycle ayant permis de valider le signal $S_{oi}$, l'amplitude $A_a$ de ce signal $S_a$ est effacée de la mémoire du micro-ordinateur 28 au cours de la boucle de programme F22 à F31, de sorte que la liste de signaux $S_{oo}$ à $S_{oi}$ indiquée ci-dessus ne comprend pas ce signal parasite $S_a$.

Comme on peut le voir sur la figure 4, le sous-programme "$K_{oi}$?" constituant chacune des étapes F19 et F27 consiste à mettre en mémoire l'amplitude $A_i$ du signal $S_{oi}$, à la comparer avec le seuil $W_i$ et à incrémenter l'indice i d'une unité si l'on a $A_i > W_i$. Dans le cas où on a $A_i \leq W_i$, l'indice i reste inchangé et un NON apparaît à la sortie du sous-programme "$K_{oi}$?".

Revenant à la figure 2, on voit que si à la suite de la validation d'un signal $S_a$, aucun signal $S_{oi}$ n'a pu être validé pendant un laps de temps de 1,6 secondes, un OUI apparaît à la sortie de l'étape F22. Ce OUI entraîne, entre autres, une diminution de l'indice i d'une unité à l'étape F26 (figure 3). Il en résulte que, au cours de l'étape F27 "$K_{oi}$?", on utilisera comme valeur de seuil $W_i$ non pas celle qui a été déterminée à l'apparition du dernier signal validé, mais la valeur précédente. De plus, l'amplitude $A_i$ du nouveau signal $S_{oi}$ validé au cours de cette étape F27 sera inscrite dans la case de mémoire d'indice i à la place de l'amplitude $A_a$ du signal parasite $S_a$ qui y était enregistrée. Il en résulte un effacement de l'information correspondant à cette amplitude $A_a$.

Bien entendu, la grandeur $G_i$ pourrait être différente de la valeur $A_{max.i}$ ou de la valeur $A_i$. Ainsi, on pourrait utiliser comme grandeur $G_i$ la moyenne arithmétique des amplitudes des signaux de la liste $S_{oo}$ à $S_{oi}$ définie ci-dessus.

Sur la figure 9, on a représenté par des traits continus verticaux les signaux S dus à des sons de Korotkoff, et par des traits verticaux discontinus les signaux S parasites dus à d'autres phénomènes. On peut voir sur la figure 9 que le signal $S_{o1}$, qui est dû à un son de Korotkoff, est suivi de près par un signal de son parasite $S_{o2a}$ d'amplitude nettement supérieure à celle du signal $S_{o1}$, et ne correspondant pas à un son de Korotkoff.

L'apparition du signal $S_{o2a}$ provoque une décompression de la chambre 3 de la quantité $\Delta P$ (étape F18). Cependant, le signal $S_{o2a}$ ayant une amplitude $A_{2a}$ anormalement élevée, il entraîne la fixation du seuil de détection à une valeur $W_{3a}$ anormalement élevée. Comme on peut le voir sur la figure 9, cette valeur de seuil $W_{3a}$ ne permet pas de valider le signal $S_{o2b}$ suivant. Il en résulte que 1,6 secondes après l'apparition du signal $S_{o2a}$, on n'a toujours pas pu valider un signal S, ce qui entraîne l'apparition d'un OUI à la sortie de l'étape F22. Ce OUI commande l'accomplissement des étapes suivantes du programme du micro-ordinateur 28.

Les étapes F24 à F30 constituent une partie de programme selon laquelle on effectue une décompression de la chambre 3 (étape F25), on donne au seuil de validation sa valeur précédente $W_2$, et on regarde si, avec ce seuil qui est nettement inférieur au seuil $W_{3a}$, on peut valider un signal S survenant moins de 1,6 secondes après la dernière décompression effectuée à l'étape F25. Dans l'exemple représenté sur la figure 9, un signal suivant $S_{o2}$ est validé en

utilisant le seuil $W_2$ et l'amplitude $A_2$ de ce signal est mémorisée à la place de l'amplitude $A_{2a}$ du signal $S_{o2a}$ de sorte que l'information de cette amplitude $A_{2a}$ est effacée.

Comme on peut le voir sur la figure 3, le OUI de l'étape F27 dû à la détection du signal $S_{o2}$ ne permet pas de redéclencher directement la boucle de programme F17 à F22 représentée sur la figure 2, des étapes intermédiaires F29 à F31 étant prévues entre la sortie OUI de l'étape d'interrogation F27 et l'entrée de l'étape F20.

Les étapes F30 et F31 constituent un sous-programme qui est représenté en détail à la figure 7. Comme on peut le voir sur cette figure, ce sous-programme consiste à effectuer une décompression de la chambre 3 à l'apparition du signal $S_{o2}$, et à effectuer un test consistant à vérifier si le signal $S_{o2}$ est suivi ou non par un autre signal S ayant une amplitude dépassant le seuil $W_3$ calculé à l'étape F29 en fonction de la grandeur $G_2$, et si cet autre signal S se produit moins de 1,6 secondes après le signal $S_{o2}$. Selon l'exemple représenté sur la figure 9, le signal $S_{o2}$ est effectivement suivi à moins de 1,6 secondes par un autre signal $S_{o3}$ dont l'amplitude $A_3$ dépasse le seuil $W_3$. Cette condition étant satisfaite, un OUI apparaissant à la sortie de l'étape d'interrogation F31 permet, par la liaison F32, de redéclencher la boucle de progamme F17 à F22 déjà décrite plus haut, ce qui provoque notamment une mise en mémoire de la pression p (étape F21).

Comme on peut le voir sur la figure 9, après la validation du signal $S_{o7}$, le seuil de détection est fixé à une valeur $W_8$ trop élevée pour permettre la validation du signal $S_{o8b}$ suivant. Il en résulte que durant le laps de temps de 1,6 secondes suivant l'apparition du signal $S_{o7}$, on n'a pas pu valider de signal S, de sorte qu'un OUI apparaît à la sortie de l'étape F22, qu'une décompression est effectuée à l'étape F25 1,6 secondes après l'apparition de ce signal $S_{07}$ et que l'indice i est diminué d'une unité à l'étape F26.

Cette diminution de l'indice i fait que le seuil de validation reprend la valeur $W_7$ qu'il avait avant l'apparition dudit signal $S_{07}$. Néanmoins, contrairement à ce qui s'est passé pour les signaux $S_{02}$ et $S_{03}$, le seuil $W_7$ ne permet pas, lui non plus, de valider un signal S dans un délai de 1,6 secondes. En conséquence, une seconde décompression est effectuée 1,6 secondes après la décompression précédente. Aucun signal S n'étant validé avec le seuil $W_7$, 1,6 seconde après cette deuxième décompression, un OUI apparaît à la sortie de l'étape F33; ce OUI déclenche l'affichage (étape F34) de la dernière valeur de la pression p mise en mémoire à l'étape F21 ainsi que l'affichage de la pression mise en mémoire au cours de l'étape F15. Ces deux pressions constituent les pressions diastolique respectivement systolique du sujet examiné.

Le OUI de l'étape F33 déclenche aussi la décompression finale de la chambre 3 (étape F35).

Il ressort de ce qui a été dit plus haut que, à chaque validation d'un signal $S_{0i}$, le seuil de validation est fixé à une nouvelle valeur $W_{i+1}$ qui est une fonction croissante f d'une grandeur $G_i$ qui est elle-même fonction de l'amplitude d'au moins un signal S validé antérieurement. Selon l'exemple présentement décrit et représenté sur la figure 8, $G_i$ est l'amplitude $A_{max.i}$ du plus grand des signaux $S_{00}$ à $S_{0i}$ validés au moment de la détermination dudit nouveau seuil, ces signaux sauf le dernier $S_{0i}$ ayant en outre chacun été identifiés comme ne provenant pas d'un son parasite par les étapes de programme F19 et F22 à F31.

Sur la figure 10, on a représenté le cas où un signal parasite $S_{o8a}$ d'amplitude élevée apparaît plus de 1,6 secondes après l'apparition du dernier signal validé $S_{o7}$. Ce signal $S_{o8a}$ est identifié comme provenant d'un son parasite par les étapes de test F30 et F31, car il entraîne la fixation du seuil à une valeur $W_{8a}$ trop élevée pour permettre la validation dans un délai de 1,6 secondes du signal suivant $S_{o8c}$ qui d'ailleurs, n'aurait pas pu être validé avec le seuil $W_7$ dû au signal $S_{o7}$. Un NON apparaît donc à la sortie de l'étape F31, de sorte que 1,6 secondes après, un OUI à la sortie de l'étape F33 entraîne l'affichage en tant que pression diastolique de la dernière pression p mise en mémoire à l'étape F21.

REVENDICATIONS

1. Procédé de mesure de la pression sanguine d'un sujet à l'aide d'un brassard (1) fixé sur un membre dudit sujet et comportant une chambre déformable (3) contenant un gaz à une première pression supérieure à la pression systolique du sujet, consistant à faire décroître la pression (p) régnant dans la chambre (3) à partir de ladite première pression jusqu'à une seconde pression, et à mesurer la pression (p) régnant dans ladite chambre durant cette décroissance de pression, caractérisé par le fait qu'il consiste à faire décroître ladite pression de façon discontinue depuis ladite première pression jusqu'à ladite seconde pression en effectuant des décompressions de même amplitude ($\Delta p$) espacées dans le temps les unes des autres.

2. Procédé selon la revendication 1, consistant en outre à détecter les sons émis dans une zone contenant ledit brassard durant ladite décroissance de pression, et à valider chaque son lorsque son intensité est supérieure à un seuil déterminé, caractérisé par le fait qu'il consiste à faire décroître ladite pression en effectuant lesdites décompressions à intervalles de temps réguliers pendant une première phase durant laquelle aucun desdits sons ne peut être validé, et, pendant une deuxième phase, en effectuant une décompression en réponse à chaque son validé.

3. Procédé selon la revendication 2, caractérisé par le fait qu'il consiste à ne commencer ladite deuxième phase qu'après avoir validé un nombre prédéterminé de premiers sons, ces premiers sons définissant une troisième phase, intermédiaire entre lesdites première et deuxième phase, et à maintenir ladite pression à une valeur constante durant cette troisième phase.

4. Procédé selon la revendication 2, caractérisé par le fait que, durant ladite deuxième phase, ledit seuil déterminé utilisé pour valider un son est une fonction croissante ($\alpha$) d'une grandeur (G) déterminée en dépendance de l'intensité d'au moins un son validé antérieurement.

5. Procédé selon la revendication 4, caractérisé par le fait que ladite grandeur est l'intensité du dernier son d'une suite de sons validés avant le son à valider.

6. Procédé selon la revendication 4, caractérisé par le fait que ladite grandeur est l'intensité du son le plus intense d'une suite de sons validés avant le son à valider.

7. Procédé selon la revendication 4, caractérisé par le fait que ladite grandeur (G) est la moyenne arithmétique des intensités des sons d'une suite de sons validés avant le son à valider.

8. Procédé selon l'une des revendications 4 à 7, caractérisé par le fait que si, durant ladite deuxième phase, aucun son n'a été validé pendant un laps de temps prédéterminé en utilisant ledit seuil déterminé, ce seuil est remplacé par le seuil avec lequel la dernière validation d'un son a été effectuée.

9. Procédé selon la revendication 8, caractérisé par le fait qu'il consiste à identifier comme son parasite le dernier son validé avant ledit laps de temps si, à la suite dudit remplacement de seuil, au moins un son suivant a été validé.

10. Procédé selon la revendication 9, caractérisé par le fait que ladite suite de sons comprend tous les sons validés durant ladite deuxième phase avant ledit dernier son et n'ayant pas été identifiés comme sons parasites.

Fig.1

**F1** DEBUT PROGRAMME PRINCIPAL

**F2** P O M P A G E

**F3** P STABLE ? — NON / OUI

**F4** INITIALISATION 1,6 SEC.

**F5** SEUIL = Wo K ? — OUI / NON

**F6** 1,6 SEC. — NON / OUI

**F12**

**F8** DECOMPRESSION D'UN PAS

**F9** INITIALISATION 1,6 SEC.

**F10** SEUIL = Wo K ? — OUI / NON

**F11** 1,6 SEC.? — NON / OUI

**F7** AFFICHAGE SIGNE INFORMATIF

**F13** VALIDATION 1er K

**F14** VALIDE ? — NON / OUI

**F15** MISE EN MEMOIRE DE P SYSTOLIQUE

**F16** i = o

**F17** INITIALISATION 1,6 SEC.

**F18** DECOMPRESSION D'UN PAS

**F21** MISE EN MEMOIRE DE p

**F20** CALCUL ET MISE EN MEMOIRE DU SEUIL

**F19** SEUIL = $W_i$ $K_{oi}$ ? — OUI / NON

**F22** 1,6 SEC. ? — NON / OUI

**F32** Y

X

*Fig.2*

X ●

Y ●

— F32

**F23** INITIALISATION COMPTEUR DE PAS DE DECOMPRESSION

**F24** INITIALISATION 1,6 SEC.

**F25** DECOMPRESSION D'UN PAS

**F26** $i = i - 1$

**F27** SEUIL = $W_i$ $K_{oi}$ ? — OUI

NON

**F28** 1,6 SEC. — NON

OUI

**F33** 2 PAS ? — NON

OUI

**F34** AFFICHAGE DE LA PRESSION SYSTOLIQUE ET DE LA DERNIERE PRESSION p MISE EN MEMOIRE

**F35** DECOMPRESSION FINALE

F I N

**F31** VALIDE ? OUI — NON

**F30** VALIDATION K

**F29** CALCUL ET MISE EN MEMOIRE DU SEUIL

*Fig.3*

0079306

4/6

**Fig.4**

SEUIL = $W_i$ $K_{oi}$ ? — OUI = 

NON

ACQUISITION DE A = AMPLITUDE D'UN SON $K_{oi}$ (SON DE K. OU AUTRE)

$A_i > W_i$ ? — OUI

NON

i = i+1

OUI

DEBUT DECOMPRESSION D'UN PAS

OUVERTURE VANNE

PRESSION DIMINUEE DE $\Delta P$? — NON

OUI

FERMETURE VANNE

RETOUR PROGRAMME PRINCIPAL

**Fig.5**

DEBUT VALIDATION 1er K

INITIALISATION COMPTEUR DE SONS

INITIALISATION 1,6 SEC.

SEUIL = $W_o$ K ? — OUI

NON

1,6 SEC. ? — NON

OUI

VALIDE = FAUX

2 SONS ? — NON

OUI

VALIDE = VRAI

RETOUR PROGRAMME PRINCIPAL

**Fig.6**

0079306

5/6

**DEBUT VALIDATION K**

INITIALISATION 1,6 SEC.

DECOMPRESSION D'UN PAS

$SEUIL = W_i$
$K_{oi}$ ? — OUI

NON

1,6 SEC. ?

NON

OUI

VALIDE = FAUX

VALIDE = VRAI

RETOUR PROGRAMME PRINCIPAL

*Fig.7*

**DEBUT CALCUL ET MISE
EN MEMOIRE DU SEUIL**

LIRE $A_{max.i-1}$

$A_i > A_{max.i-1}$ — NON

OUI

$A_{max.i} = A_i$

$A_{max.i} = A_{max.i-1}$
$W_{i+1} = W_i$

CALCUL DE $W_{i+1} = f (A_{max.i})$

MISE EN MEMOIRE
DE $W_{i+1}$

RET. AU PROGRAMME PRINCIP

*Fig.8*

Fig.10

Fig.9

9/6

0079306